# EUROPEAN PATENT SPECIFICATION

(11) **EP 1 708 774 B1**
(45) Date of publication and mention of the grant of the patent: **11.06.2008**
(21) Application number: 04730130.4
(22) Date of filing: 28.04.2004
(51) Int. Cl.: A61M 16/04

(54) **RESPIRATORY TUBE HAVING A CUFF WITH A CONSTRICTION ZONE**
BEATMUNGSTUBUS MIT EINER BALLONMANSCHETTE MIT EINEM VERENGTEN ABSCHNITT
TUBE RESPIRATEUR POURVU D'UN BALLONNET A ZONE DE CONSTRICTION

(30) Priority: 18.12.2003 NL 1025062
(43) Date of publication of application: 11.10.2006
(73) Proprietor: Masterflex AG, 45841 Gelsenkirchen (DE)
(72) Inventor: LADRU, Pietronella Christina, Curaçao (AN); VAN DEN BERG, Paulus Cornelis Maria, 1103 PR Amsterdam (NL)
(74) Representative: Behrendt, Arne
(86) International application number: PCT/NL2004/000282
(87) International publication number: WO 2005/058402

(56) References cited:
- EP-A- 0 865 798
- EP-A- 0 884 060
- EP-A- 1 005 877
- WO-A-96/27404
- WO-A-03/020340
- DE-A- 19 848 429
- DE-U- 29 806 178
- US-A- 2 099 127
- US-A- 3 810 474

## Description

The present invention relates to a respirator for a person or animal comprising a tube assembly that is equipped to be fed via the mouth and the pharynx towards the trachea and an inflatable cuff - which is also called balloon - that is provided at the distal end of the tube assembly, the cuff being equipped to form a seal between the wall of the tube assembly and a wall of the pharynx when it is in the inflated state, the tube assembly having a first tube part and the tube assembly having a length suitable for bringing the distal end of the first tube part to the entry to the trachea while the proximal end of the tube assembly is outside the mouth.

Such a respirator, which is regularly used in the medical care sector, is known, for example from International Patent Application WO 95/06492.

The said patent application describes a respirator that comprises, inter alia, a tube (12) and an inflatable cuff (14). From the description of the figure in said WO 95/06492 it can be seen that the pharynx is closed off from the tube wall with the aid of the cuff (14). It can furthermore be seen that the distal end of the tube (12) extends into the lower part of the pharynx, just above the epiglottis (26). In the inflated state, the cuff (14) is provided with a projection (28) on the front of the cuff so as to keep the respirator behind the tongue. A limitation of this and other known respirators is that the access to the oesophagus is not closed off, or at least is not closed off well. If there is communication between the stomach and the lungs, stomach contents can run into the lungs via the hypopharynx during respiration. Especially in the event of a build-up of pressure by gases in the stomach or in the case of belching, the cuff according to the state of the art can be pushed upwards, as a consequence of which a communication between stomach and lungs can be produced underneath the cuff.

European Patent EP 0 971 765, considered as the closest prior art for the present invention, describes a similar type of respirator where an inflatable cuff (2) is likewise used to provide a closure between tube (1) and pharynx. One advantage of this respirator compared with that from WO 95/06492 is that the tube (1) is provided at the distal end with a strip (3) that is intended to be fitted in the top end of the oesophagus. This strip (3) prevents the distal end of the tube being inserted into the oesophagus and thus, for example, being able to damage the vocal chords. The known respirators also inadequately prevent stomach contents passing into the trachea and lungs.

DE 298 06 178 U1 describes a larynx mask with an oval part to be introduced into the pharynx comprising a pocket for the distal part of a stick, which helps in positioning of the mask. However, this document does not disclose a distal cuff part intended or suitable to close off the oesophagus.

The aim of the present invention is to provide a respirator that effectively prevents stomach contents passing into the trachea and lungs by tackling the effects favouring a build-up of pressure by gases from the stomach. According to the invention this problem can be approached in essentially two ways, which in particular are employed simultaneously, but which each individually also offer major advantages.

The aim of the present invention is achieved according to the first route by providing a respirator according to Claim 1 and is achieved according to the second route by providing a respirator according to Claim 7. Both routes have in common that they provide means with which the influence of pressure effects as a consequence of pressure build-up by gases in the stomach is kept away from the trachea.

According to the first route by means of which the aim of the invention is achieved, the cuff has a distal cuff part intended to extend into the oesophagus and to close off the oesophagus when in the inflated state, and the distal cuff part has a constriction zone which, in the inflated state, provides a constriction in the distal cuff part. If inflated, the cuff thus closes off not only against the wall of the pharynx but also against the peripheral wall of the upper part of the oesophagus. This has the significant advantage that the oesophagus is completely closed off, as a result of which the ingress of accumulated fluid or of stomach contents into the trachea can be very reliably prevented. The constriction - that is to say the distal cuff part is thicker at the distal end than at the proximal end where the constriction is located - furthermore ensures fixation because sphincter muscle tissue of the oesophagus will engage on this and will thus hold the cuff firmly and will fix it in the longitudinal direction of the oesophagus. As is known, the sphincter muscle tissue of the oesophagus has a peristaltic action effective in the direction of the stomach. This peristaltic action not only promotes retention of the entire construction but furthermore provides a very good and reliable sealing effect in combination with the distal part of the cuff. It is, in particular, this additional sealing effect that ensures that excess pressure effects of gases from the stomach are kept away from the trachea.

With regard to the distal part of the cuff it is advantageous according to the invention if the constriction zone has a length of 1 to 4 cm, in particular 1.5 to 3 cm, such as approximately 2 cm. Since in practice the constriction zone will form part of a one-part balloon body from which the entire cuff is made and sharp transitions are undesirable in such a balloon body in connection with use in the human body, such as with a view to the peristaltic action, it will be clear that the length of the constriction zone - which length is considered between proximal and distal in the direction of extent - will have no sharp boundaries in practice. Incidentally, the same also applies in respect of the length of the part of the distal part of the cuff that is located distally from the constriction zone and will be discussed below.

In connection with accommodation in the oesophagus and sealing on the inside of the oesophagus, it is furthermore advantageous according to the invention if the distal part of the cuff has, distal to the constriction zone, a section that is tubular in the inflated state. According to the invention, this tubular section preferably has a length in the range from 1.5 to 10 cm. It is pointed out that, certainly in the case of adults, the length of 10 cm can easily be exceeded because the oesophagus from the pharynx to the entry to the stomach is significantly longer. However, placing the respirator in the patient via the mouth will become more difficult as the tubular section becomes longer. In order to ensure that the tubular section has a good seal on the inside of the oesophagus, it is preferable according to the invention if the length of the tubular section is longer than approximately 2 cm and is preferably longer than approximately 3.5 cm. In order to prevent the tubular section becoming too long and also partly to ensure that the seal on the inside of the oesophagus is still very good, it is preferable according to the invention if the length of the tubular section is shorter than approximately 8 cm and is preferably approximately 8 cm or shorter.

In the general sense, it is pointed out with regard to the dimensions of all components of the respirator that these will be patient-dependant to some extent. The components of the respirator will be able to be significantly smaller in the case of children than in the case of adults. In practice, the respirator will be made available [lacuna] a number of standard sizes. The specialist giving treatment can then establish which respirator is used for which patient.

As stated, the second route, to be discussed below, can also be employed independently of the first route, although less advantageously.

The second tube part used in the second route makes it possible to allow excess pressure caused by gases or belching to escape via the second tube part. This appreciably reduces the risk that stomach contents can pass into the lungs. After all, if stomach contents rise, these, or at least the pressure, will be able to escape through the second tube part, so that the rising stomach contents will not be forced past the cuff. An additional advantage is that a lower inflation pressure on the cuff can suffice, so that the inflated cuff can remain in the patient for much longer without too much damage to tissue on which the cuff is pressing. After all, a relatively low air pressure in the inflatable cuff suffices to close off the pharynx, which reduces the pressure on the blood vessels, as a result of which these are not pressed shut and circulation through the mucus membrane remains good and necrosis of the wall of the pharynx is prevented, as has been stated. This means that the respirator according to the invention can remain in the patient for much longer than the devices known from the state of the art. In the case of the respirator according to the invention, an internal pressure of 20 to 30 cm water pressure can suffice for the cuff, it then being possible to respirate at approximately 50 cm water pressure. Respirators according to the state of the art require an internal pressure for the cuff of approximately 60 cm water pressure for adequate closing-off, whilst in addition only a maximum pressure of approximately 30 cm water pressure is permissible for respiration. A further advantage of the second tube is that this can be used for inserting instruments through it, for example for intervention in or inspections of the oesophagus or the stomach. Instruments that can be considered are diverse probes, a small camera, etc. Furthermore, the second tube can be used for feeding fluids, such as food, to the stomach.

With a view to a good seal, it is advantageous according to the invention if the cuff is provided around the distal end of the tube assembly and that the distal end of the second tube part extends through the cuff in a sealed manner.

According to an advantageous embodiment of the invention, the internal part of the first tube part is separate from the internal part of the second tube part. The clear advantage is that as a result mixing of gases and fluids that run through the tube parts is prevented at all times.

According to the invention it is advantageous if the second tube part of the respirator has a non-circular cross-section, such as an oval cross-section. If a probe or another instrument with a non-circular or a likewise oval cross-section is inserted in such a tube, rotations about the longitudinal axis of the probe or the instrument can essentially be prevented, so that the probe or the instrument can be positioned more accurately.

According to the invention it is furthermore advantageous if the inside of the second tube part has a circular cross-sectional shape. A circular shape is simpler from the standpoint of production engineering and conventional probes also usually have an essentially circular cross-sectional shape.

According to an advantageous aspect of the invention, a flexible stiffener that extends as far as the tip of the distal part of the cuff is provided in the distal part of the cuff. This aspect makes it easier to insert the cuff in the entry to the oesophagus. Moreover, it is possible to construct the stiffener in such a way that it becomes impossible accidentally to insert the cuff and the second tube part in the trachea. The latter is possible, in particular, by making the first tube part curved and providing the stiffener, running from the distal end of the first tube part in the extension thereof, on the side of the outside bend of said curvature. Furthermore, hardly any or no special training is required for introducing a respirator with this configuration.

With a view to a good seal in the pharynx, it is advantageous if the cuff is so fitted asymmetrically on the tube assembly and also has such a shape that, when the proximal part of the cuff is in the inflated state in the pharynx, the proximal part of the cuff essentially fills the pharynx and the pushes the distal orifice of the first tube part in front of the entry to the trachea.

With a view to easily fitting the respirator according to the invention in the patient, it is advantageous if the tube assembly has a curved shape and if the distal orifice of the first tube part, viewed in the radial direction, is provided on the inside of the second tube part.

The present invention also relates to a combination of a respirator as described above and a probe, the probe being suitable for insertion through the second tube. In the case of some operations it will be advantageous if it is possible at one and the same time to respirate and to administer or draw off food or other fluids via the probe.

The respirator according to invention is, in particular, very suitable for use in intensive care, in anaesthesia, in accident and emergency, in resuscitation and also in trauma services. Both medical staff, paramedics and also untrained staff are able to position the respirator according to the invention safely in a patient.

The mode of operation and the use of the respirator according to the present invention will be explained on the basis of the following figures, which show an illustrative embodiment and in which;
Figure 1 shows a side view of a first embodiment of the respirator according to the invention, where the cuff has been inflated slightly;
Figure 2 shows a side view of the respirator according to the present invention in the position introduced in a patient;
Figure 3 shows a cross-section according to III-III in Figure 1; and
Figure 4 shows a cross-section according to IV-IV in Figure 1; and
Figure 5 shows a side view of a second embodiment of the respirator according to the invention.

The figures shows a tube assembly 33 with a first tube part, the respiration tube 1, which continues as far as a distal orifice end 14, and with a second tube part 2. The first and second tube parts 1, 2 are, at least partially, accommodated in a common sheath 3.

An inflatable cuff 4, which is also called balloon, is fixed at the distal end of sheath 3 in a sealed manner.

The second tube part 2 continues in the distal direction with respect to the distal end 14 of the respirator tube 1.

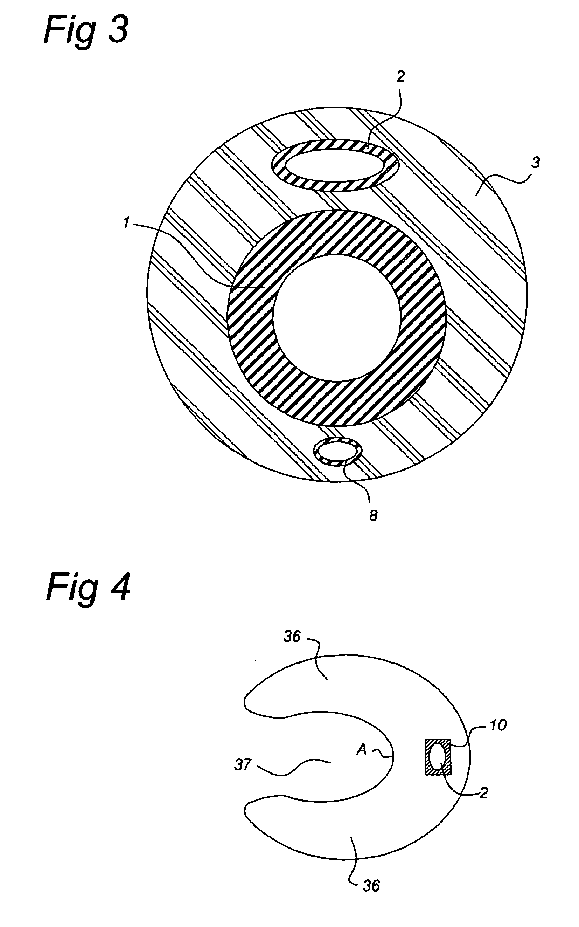

The distal end 5 of the second tube part 2 protrudes through the inflatable cuff 4 in a sealed manner. In the cuff 4 the second tube part is in the form of a flexible stiffener 10. The function of the stiffener 10 will be explained further below. Here, the internal cross-section of the second tube is non-circular, such as oval (see Figure 3). A probe with a matching non-circular or oval cross-section, which is inserted in the second tube part, will consequently be prevented from rotating, which is advantageous when manipulating the probe.

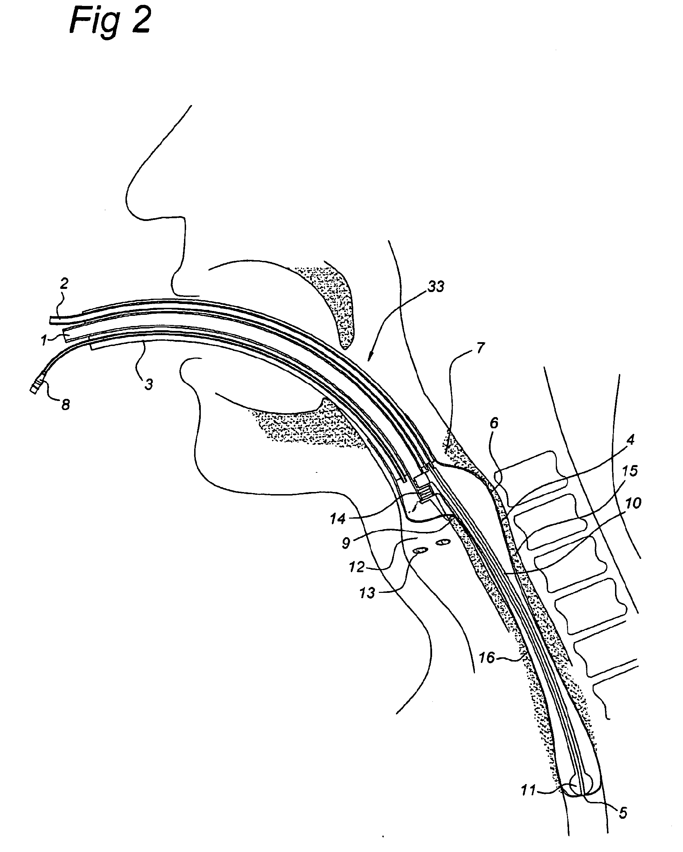

In the use position of the respirator in Figure 2 it can be seen that, in the inflated state, the cuff 4 completely closes off the oesophagus 6.

Furthermore, in the inflated state, the cuff 4 forms a seal between the pharynx 7 and the tube assembly 33.

The cuff can be inflated via a channel 8 that runs through the sheath 3 from the proximal end and that opens with the proximal end in the interior of the cuff 4. The cuff is thus inflatable, as indicated by arrow 40. After use, the cuff can also be emptied by suction via channel 8.

A stiffener 10 is provided at the distal end of the respirator tube 1. The stiffener 10 is so positioned that it guides the tube assembly 33, when this is inserted into the patient, in such a way that the orifice 14 of the first tube part I comes in front of the opening of the trachea 12. The stiffener 10 has a number of functions:
- the stiffener serves to direct the respirator in the right direction when inserting;
- once the respirator is in position, the stiffener serves to fix it in this position so that movements of the head (turning, flexion) do not lead to dislocation of the respirator;
- the stiffener serves to guide the cuff, that runs over the stiffener, into the oesophagus;
- during insertion, the stiffener, which can consist of a flexible hollow tube, is pushed over the rear wall of the pharynx and thus automatically passes into the oesophagus. Anaesthesiologists know that when an endotracheal tube is inserted this always ends up in the oesophagus. The aim of the technique for inserting an endotracheal respiration tube is, therefore, to prevent this. After all, the principle of the endotracheal tube is based on the fact that this is introduced into the trachea so as to be able to breathe or respirate through this. The stiffener thus makes use of the fact that when a hollow tube with the correct curvature is introduced this tube automatically ends up in the oesophagus.

The strip 9 at the distal end 14 of the respiration tube 1 prevents the cuff 4 shutting off the trachea on inflation. With this arrangement the strip 9 can bear on the septum between oesophagus and trachea and can partially extend into the oesophagus.

The function of the stiffener 2 with regard to the correct positioning of the respirator can be seen in Fig. 2. The round end section 11 of the stiffener is intended to counteract damage to the mucus membrane and also to the cuff and extends a few centimetres into the oesophagus.

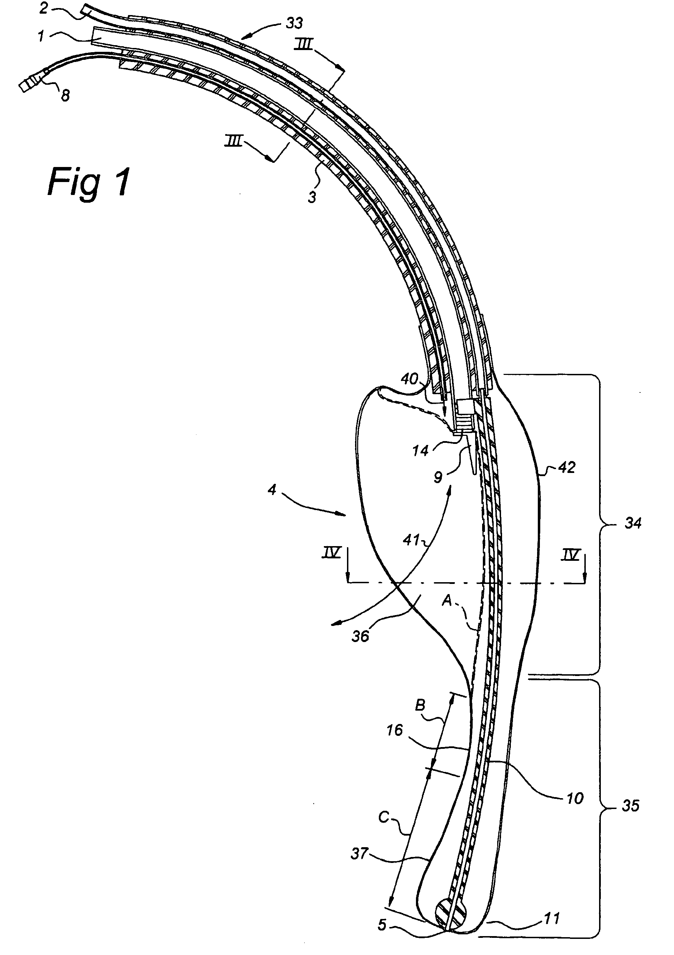

The cuff 4 has a proximal cuff part 34 and a distal cuff part 35. In a cross-sectional plane IV-IV (see Fig. 4), the proximal cuff part 34 has a U-shaped cross-sectional shape with two arms 36, pointing to the left in Figure 1 and 4, with a space 37 between them in which orifice end 14 and strip 19 are located. This space 37 is delimited at the rear by a wall section A of the cuff. Double-headed arrow 41 indicates, diagrammatically, respiration via the first tube part 1.

The distal part 35 of the cuff has a constriction zone 16 of length B, in which, when the cuff has been inflated and placed in a patient, sphincter muscle tissue on the oesophagus 15 engages. Distal to the constriction zone 16, the distal part 35 of the cuff has a tubular section 37 of length C.

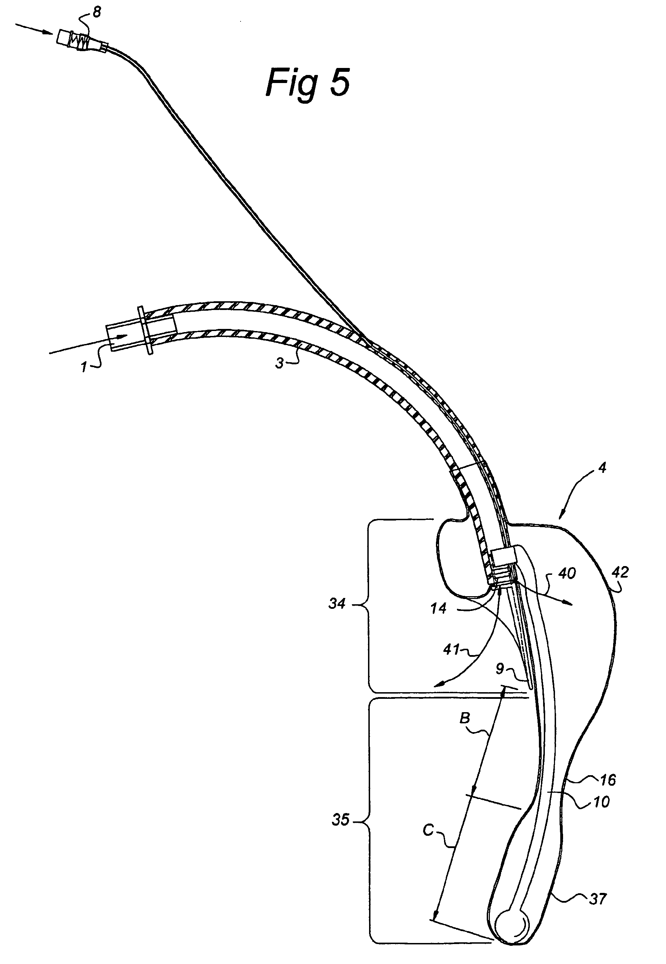

Figure 5 shows a second embodiment of a respirator according to the invention that is somewhat modified compared with Fig. 1. Reference numerals and symbols corresponding to the earlier figures have been used in Figure 5 for corresponding components.

In the embodiment according to Fig. 5, the second tube part for venting and/or access to the oesophagus has been omitted. However, it will be clear to a person skilled in the art that this second tube part can easily be provided, in a manner corresponding to Fig. 1, in the embodiment according to Figure 5. The stiffener 10 can be made hollow and a small tube can be connected thereto, correspondingly as in the case of the inflation channel 8. This small tube, which in particular will be flexible, can then be guided out of the tube 3 to the outside, just above the proximal part of the cuff 4.

A further difference compared with the embodiment according to Fig. 1 is that the strip 9 is longer, that the constriction 16 is more pronounced and that the proximal part 34 has more volume at the rear 42.

In Fig. 5 the cuff is shown in a slightly inflated state, in which the cuff just bulges but is not yet taut. As an indication of dimensions, it is pointed out that the height of the proximal part 34 of the cuff can be approximately equal to the length of the distal part 35 of the cuff, both dimensions then being measured in the direction as indicated by the brackets.

An additional advantage is that, because of its simple construction and the relatively low cost price, the respirator according to the invention can be used as a disposable article.

The distal part of the cuff according to the invention that is in the oesophagus offers a number of advantages:
- by this means this respirator is the first that has an inflatable balloon that continues deep into the oesophagus. This embodiment has the advantage that even better fixing of the respirator is produced;
- furthermore, this embodiment of the balloon in the oesophagus ensures that in the event of slight dislocation this does not immediately produce inadequate closure of the oesophagus, as is the case with the respirators according to current technology;
- during vomiting, contents of the stomach are fed upwards via the oesophagus and the muscles in the wall of the oesophagus contract. The result is that the pressure in the oesophagus increases substantially. In the case of the respirators according to current technology, this increase in pressure causes these devices to be pushed upwards and no longer guarantee adequate closure of the oesophagus. The effect described will also arise with respirators with a venting channel. After all, at a given point in time the vomit will pass into this venting channel and shut it off, as a result of which the pressure build-up in the oesophagus still takes place. With the present system the closure of the oesophagus by the balloon remains adequate even in the case of pressure build-up in the oesophagus. This is achieved because the balloon continues deep into the oesophagus. As a result of pressure build-up in the oesophagus, the balloon will be firmly fixed in the oesophagus and will not be pushed upwards. The pressure in the cuff will increase, as a result of which, because of the wedge shape of the cuff, the balloon is more likely to be pushed deeper into the pharynx and thus closes off better. This better closing off is partly caused because the air in the oesophagus part of the balloon passes into the pharynx part of the balloon. This also explains why an adequate seal can be achieved with relatively low pressures in the balloon. As a result of the mechanism described, the pressure in the balloon increases at the point in time when this is needed, i.e. during vomiting. The aim with the respirator according to current technology is to obtain an adequate seal by inflating the cuff to a very great extent, which is harmful for the wall of the pharynx.

An important reason why a good airtight closure can be achieved with such low pressures lies in the fact that, in the inflated state, the cuff section that is in the pharynx assumes precisely the shape of the pharynx, as a result of which a large contact surface between cuff and pharynx is achieved, which is not the case with other respirators according to the current state of the art. A good airtight seal is not given in the medical scientific literature.

The embodiment of the respirator drawn in the figures and discussed has been indicated merely by way of example.

## Claims

1. Respirator for a person or animal comprising a tube assembly (33) that is intended to be fed via the mouth and the pharynx (7) towards the trachea (12) and an inflatable cuff (4) that is provided at the distal end of the tube assembly (33), the cuff (4) being equipped to form a seal between the wall of the tube assembly (33) and a wall of the pharynx (7) when it is in the inflated state, the cuff (4) having a distal cuff part (35) intended to extend into the oesophagus (6) and, in the inflated state, to close off the oesophagus (6), the tube assembly (33) having a first tube part (1) and the tube assembly (33) having a length suitable for bringing the distal end (14) of the first tube part (1) to the entry to the trachea (12) while the proximal end of the tube assembly (33) is outside the mouth, **characterised**
**in that** the distal cuff part (35) has a constriction zone (16) which, in the inflated state, provides a constriction (16) in the distal cuff part (35) and that the distal cuff part (35) is thicker at the distal end than at the proximal end where the constriction (16) is located.

2. Respirator according to Claim 1, **characterised in that** the constriction zone (16) has a length of 1 to 4 cm, in particular 1.5 to 3 cm, such as approximately 2 cm.

3. Respirator according to one of the preceding claims, wherein the distal part (35) of the cuff (4) has, distal to the constriction zone (16), a section (37) that is tubular in the inflated state.

4. Respirator according to Claim 3, **characterised in that** the tubular section (37) has a length of 1.5 to 10 cm.

5. Respirator according to Claim 3 or Claim 4, wherein the length of the tubular section (37) is longer than approximately 2 cm and is preferably longer than approximately 3.5 cm.

6. Respirator according to one of Claims 3-5, wherein the length of the tubular section (37) is shorter than approximately 8 cm and is preferably shorter than approximately 6 cm.

7. Respirator according to one of the preceding claims, **characterised in that** the tube assembly (33) has a second tube (2) part with a length suitable for introducing the distal end (5) of the second tube part (2) into the oesophagus (6) whilst the proximal end of the second tube part (2) is outside the mouth.

8. Respirator according to Claim 7, **characterised in that** the cuff (4) is provided around the distal end of the tube assembly (33) and **in that** the distal end (5) of the second tube part (2) extends through the cuff (4) in a sealed manner.

9. Respirator according to Claim 7 or 8, **characterised in that** the interior part of the first and second tube parts (1, 2) are separate from one another.

10. Respirator according to one of Claims 7-9, **characterised in that** the inside of the second tube part (2) has a non-circular cross-sectional shape, such as an oval cross-sectional shape.

11. Respirator according to one of Claims 7-9, **characterised in that** the inside of the second tube part (2) has a circular cross-sectional shape.

12. Respirator according to one of the preceding claims, **characterised in that** a flexible stiffener (10) that extends as far as the tip of the distal part (35) of the cuff (4) is provided in the distal part (35) of the cuff (4).

13. Respirator according to Claim 8-12 in combination with at least one of Claims 7-11, **characterised in that** the second tube part (2) runs through the flexible stiffener (10).

14. Respirator according to one of the preceding claims, **characterised in that** the cuff (4), or at least a proximal part (34) thereof, is so fitted asymmetrically on the tube assembly (33) and also has such a shape that, when the proximal part (34) of the cuff (4) is in the inflated state in the pharynx (7), the proximal part (34) of the cuff (4) essentially fills the pharynx (7) and pushes the distal orifice (14) of the first tube part (1) in front of the entry to the trachea (12).

15. Respirator according to one of the preceding claims with at least one of Claims 7-11, **characterised in that** the tube assembly (33) has a curved shape and **in that** the distal orifice (14) of the first tube part (1), viewed in the radial direction, is provided on the inside of the second tube part (2).

16. Respirator according to one of the preceding claims, **characterised in that** the interior of the proximal part (34) of the cuff (4) is in fluid communication with the interior of the remainder of the cuff (4), such that, in the inflated state, the same pressure prevails throughout the cuff (4).

17. Respirator according to one of the preceding claims, **characterised in that** the part of the cuff (4) that, in the inflated state, is located in the pharynx (7) has a wedge-like shape with a greater volume proximally than distally, such that this part of the cuff (4) located in the pharynx (7), in the inflated state, pushes the respirator towards the oesophagus (6).

18. Combination of a respirator according to one of the preceding claims, including at least one of Claims 7 - 11, and a probe, such as a stomach tube, duodenum tube or feeding tube, wherein the probe is suitable for insertion through the second tube (2).

## Patentansprüche

1. Beatmungsgerät für eine Person oder ein Tier mit einer Röhrenanordnung (33), die dazu vorgesehen ist, über den Mund und den Rachen (7) zur Luftröhre (12) geführt zu werden, und einer aufblasbaren Manschette (4), die am distalen Ende der Röhrenanordnung (33) angeordnet ist, wobei die Manschette (4) so ausgestaltet ist, dass sie eine Dichtung zwischen der Wand der Röhrenanordnung (33) und einer Wand des Rachens (7) herbeiführt, wenn sie sich im aufgeblasenen Zustand befindet, wobei die Manschette (4) einen distalen Manschettenabschnitt (35) aufweist, der dazu vorgesehen ist, sich in die Speiseröhre (6) zu erstrecken und im aufgeblasenen Zustand die Speiseröhre (6) zu verschließen, wobei die Röhrenanordnung (33) eine erste Röhre (1) aufweist und die Röhrenanordnung (33) eine Länge hat, die geeignet ist, das distale Ende (14) der ersten Röhre (1) zum Eingang der Luftröhre (12) zu bringen, während sich das proximale Ende der Röhrenanordnung (33) außerhalb des Mundes befindet, **dadurch gekennzeichnet, dass** der distale Manschettenabschnitt (35) eine Einschnürungszone (16) aufweist, die im aufgeblasenen Zustand eine Einschnürung (16) im distalen Manschettenabschnitt (35) herbeiführt, und dass der distale Manschettenabschnitt (35) an seinem distalen Ende dicker ist als am proximalen Ende, wo sich die Einschnürung befindet.

2. Beatmungsgerät nach Anspruch 1, **dadurch gekennzeichnet, dass** die Einschnürungszone (16) eine Länge von 1 bis 4 cm, insbesondere 1,5 bis 3 cm, wie z. B. ungefähr 2 cm aufweist.

3. Beatmungsgerät nach einem der vorhergehenden Ansprüche, wobei der distale Abschnitt (35) der Manschette (4) distal zur Einschnürungszone (16) einen Bereich (37) aufweist, der im aufgeblasenen Zustand röhrenförmig ist.

4. Beatmungsgerät nach Anspruch 3, **dadurch gekennzeichnet, dass** der röhrenförmige Bereich (37) eine Länge von 1,5 bis 10 cm hat.

5. Beatmungsgerät nach Anspruch 3 oder 4, wobei die Länge des röhrenförmigen Bereichs (37) länger als ungefähr 2 cm und vorzugsweise länger als ungefähr 3,5 cm ist.

6. Beatmungsgerät nach einem der Ansprüche 3 bis 5, wobei die Länge des röhrenförmigen Bereichs (37) kürzer als ungefähr 8 cm und vorzugsweise kürzer als ungefähr 6 cm ist.

7. Beatmungsgerät nach einem der vorhergehenden Ansprüche, **dadurch gekennzeichnet, dass** die Röhrenanordnung (33) eine zweite Röhre (2) aufweist mit einer Länge, die geeignet ist, das distale Ende (5) der zweiten Röhre (2) in die Speiseröhre (6) einzuführen, während sich das proximale Ende der zweiten Röhre (2) außerhalb des Mundes befindet.

8. Beatmungsgerät nach Anspruch 7, **dadurch gekennzeichnet, dass** die Manschette (4) um das distale Ende der Röhrenanordnung (33) angeordnet ist und das distale Ende (5) der zweiten Röhre (2) sich durch die Manschette (4) in dichtender Weise erstreckt.

9. Beatmungsgerät nach Anspruch 7 oder 8, **dadurch gekennzeichnet, dass** die Innenbereiche der ersten und zweiten Röhre (1, 2) voneinander getrennt sind.

10. Beatmungsgerät nach einem der Ansprüche 7 bis 9, **dadurch gekennzeichnet, dass** der Innenbereich der zweiten Röhre (2) einen nichtkreisförmigen Querschnitt wie beispielsweise einen ovalen Querschnitt aufweist.

11. Beatmungsgerät nach einem der Ansprüche 7 bis 9, **dadurch gekennzeichnet, dass** der Innenbereich der zweiten Röhre (2) einen kreisförmigen Querschnitt hat.

12. Beatmungsgerät nach einem der vorhergehenden Ansprüche, **dadurch gekennzeichnet, dass** eine flexible Versteifung (10) im distalen Abschnitt (35) der Manschette (4) angeordnet ist, die sich so weit erstreckt wie die Spitze des distalen Abschnitts (35) der Manschette (4).

13. Beatmungsgerät nach einem der Ansprüche 8 bis 12 in Kombination mit zumindest einem der Ansprüche 7 bis 11, **dadurch gekennzeichnet, dass** die zweite Röhre (2) durch die flexible Versteifung (10) verläuft.

14. Beatmungsgerät nach einem der vorhergehenden Ansprüche, **dadurch gekennzeichnet, dass** die Manschette (4) oder zumindest ein proximaler Abschnitt (34) hiervon so asymmetrisch an der Röhrenanordnung (33) angebracht ist und auch eine solche Form aufweist, dass der proximale Abschnitt (34) der Manschette (4), wenn der proximale Abschnitt (34) der Manschette (4) sich im aufgeblasenen Zustand im Rachen (7) befindet, den Rachen (7) im Wesentlichen ausfüllt und die distale Öffnung (14) der ersten Röhre (1) vor den Eingang der Luftröhre (12) drückt.

15. Beatmungsgerät nach einem der vorhergehenden Ansprüche mit zumindest einem der Ansprüche 7 bis 11, **dadurch gekennzeichnet, dass** die Röhrenanordnung (33) eine gekrümmte Form hat und die distale Öffnung (14) der ersten Röhre (1), in radialer Richtung betrachtet, auf der Innenseite der zweiten Röhre (2) angeordnet ist.

16. Beatmungsgerät nach einem der vorhergehenden Ansprüche, **dadurch gekennzeichnet, dass** das Innere des proximalen Abschnitts (34) der Manschette (4) mit dem Inneren der übrigen Manschette (4) über ein Fluid in Verbindung steht, so dass im aufgeblasenen Zustand überall in der Manschette (4) der gleiche Druck herrscht.

17. Beatmungsgerät nach einem der vorhergehenden Ansprüche, **dadurch gekennzeichnet, dass** der Teil der Manschette (4), der sich im aufgeblasenen Zustand im Rachen (7) befindet, eine Keilform mit einem größeren Volumen proximal als distal aufweist, so dass dieser Teil der im Rachen (7) platzierten Manschette (4) im aufgeblasenen Zustand das Beatmungsgerät zur Speiseröhre (6) drückt.

18. Kombination aus einem Beatmungsgerät gemäß einem der vorhergehenden Ansprüche, umfassend zumindest einen der Ansprüche 7 bis 11, und einer Sonde, wie beispielsweise eine Magenröhre, eine Zwölffingerdarmröhre oder eine Röhre für die künstliche Ernährung, wobei die Sonde zur Einführung durch die zweite Röhre (2) geeignet ist.

## Revendications

1. Dispositif respirateur pour une personne ou un animal comprenant un ensemble de tubes (33) qui est destiné à être introduit par la bouche et le pharynx (7) vers la trachée (12) et un ballonnet gonflable (4) qui est réalisé à l'extrémité distale de l'ensemble de tubes (33), le ballonnet (4) étant équipé pour former un joint d'étanchéité entre la paroi de l'ensemble de tubes (33) et une paroi du pharynx (7) lorsqu'il est à l'état gonflé, le ballonnet (4) ayant une partie de ballonnet distale (35) destinée à s'étendre dans l'oesophage (6) et, à l'état gonflé, à fermer l'oesophage (6), l'ensemble de tubes (33) ayant une première partie de tube (1), et l'ensemble de tubes (33) ayant une longueur appropriée pour amener l'extrémité distale (14) de la première partie de tube (1) à l'entrée de la trachée (12) pendant que l'extrémité proximale de l'ensemble de tubes (33) est à l'extérieur de la bouche, **caractérisé en ce que** la partie de ballonnet distale (35) présente une zone de constriction (16) qui, à l'état gonflé, réalise une constriction (16) dans la partie de ballonnet distale (35), et **en ce que** la partie de ballonnet distale (35) est plus épaisse à l'extrémité distale qu'à l'extrémité proximale où se situe la constriction (16).

2. Dispositif respirateur selon la revendication 1, **caractérisé en ce que** la zone de constriction (16) possède une longueur de 1 à 4 cm, en particulier de 1,5 à 3 cm, par exemple approximativement de 2 cm.

3. Dispositif respirateur selon l'une des revendications précédentes, où la partie distale (35) du ballonnet (4) présente, distalement à la zone de constriction (16), une section (37) qui est tubulaire à l'état gonflé.

4. Dispositif respirateur selon la revendication 3, **caractérisé en ce que** la section tubulaire (37) présente une longueur de 1,5 à 10 cm.

5. Dispositif respirateur selon la revendication 3 ou la revendication 4, où la longueur de la section tubulaire (37) est plus longue qu'environ 2 cm et est de préférence plus longue qu'environ 3,5 cm.

6. Dispositif respirateur selon l'une des revendications 3 à 5, où la longueur de la section tubulaire (37) est plus courte qu'environ 8 cm et est de préférence plus courte qu'environ 6 cm.

7. Dispositif respirateur selon l'une des revendications précédentes, **caractérisé en ce que** l'ensemble de tube (33) possède une seconde partie de tube (2) d'une longueur appropriée pour introduire l'extrémité distale (5) de la seconde partie de tube (2) dans l'oesophage (6) tandis que l'extrémité proximale de la seconde partie de tube (2) est à l'extérieur de la bouche.

8. Dispositif respirateur selon la revendication 7, **caractérisé en ce que** le ballonnet (4) est prévu autour de l'extrémité distale de l'ensemble de tubes (33), et **en ce que** l'extrémité distale (5) de la seconde partie de tube (2) s'étend à travers le ballonnet (4) d'une manière étanche.

9. Dispositif respirateur selon la revendication 7 ou 8, **caractérisé en ce que** la partie intérieure des première et seconde parties de tube (1, 2) sont séparées l'une de l'autre.

10. Dispositif respirateur selon l'une des revendications 7 à 9, **caractérisé en ce que** l'intérieur de la seconde partie de tube (2) présente une forme non-circulaire en section transversale, comme une forme ovale en section transversale.

11. Dispositif respirateur selon l'une des revendications 7 à 9, **caractérisé en ce que** l'intérieur de la seconde partie de tube (2) possède une forme circulaire en section transversale.

12. Dispositif respirateur selon l'une des revendications précédentes, **caractérisé en ce qu'**un organe de raidissement flexible (10), qui s'étend jusqu'à la pointe de la partie distale (35) du ballonnet (4), est prévu dans la partie distale (35) du ballonnet (4).

13. Dispositif respirateur selon les revendications 8 à 12 en combinaison avec au moins une des revendications 7 à 11, **caractérisé en ce que** la seconde partie de tube (2) passe à travers l'organe de raidissement flexible (10).

14. Dispositif respirateur selon l'une des revendications précédentes, **caractérisé en ce que** le ballonnet (4), ou au moins sa partie proximale (34) est montée d'une manière asymétrique sur l'ensemble de tubes (33) et possède également une forme telle que, lorsque la partie proximale (34) du ballonnet (4) se trouve à l'état gonflé dans le pharynx (7), la partie proximale (34) du ballonnet (4) remplit essentiellement le pharynx (7) et pousse l'orifice distal (14) de la première partie de tube (1) devant l'entrée à la trachée (12).

15. Dispositif respirateur selon l'une des revendications précédentes avec au moins une des revendications 7 à 11, **caractérisé en ce que** l'ensemble de tubes (33) possède une forme courbée et **en ce que** l'orifice distal (14) de la première partie de tube (1), vu dans la direction radiale, est prévu sur l'intérieur de la seconde partie de tube (2).

16. Dispositif respirateur selon l'une des revendications précédentes, **caractérisé en ce que** l'intérieur de la partie proximale (34) du ballonnet (4) est en communication fluidique avec l'intérieur du restant du ballonnet (4) de telle sorte que, à l'état gonflé, la même pression règne partout dans le ballonnet (4).

17. Dispositif respirateur selon l'une des revendications précédentes, **caractérisé en ce que** la partie du ballonnet (4) qui, à l'état gonflé, se situe dans le pharynx (7) présente une forme semblable à un coin avec un plus grand volume proximalement que distalement de sorte que cette partie du ballonnet (4) située dans le pharynx (7), à l'état gonflé, pousse le dispositif respirateur vers l'oesophage (6).

18. Combinaison d'un dispositif respirateur selon l'une des revendications précédentes, incluant au moins une des revendications 7 à 11, et une sonde, comme une sonde gastrique, une sonde duodénum ou une sonde gastrique d'alimentation, où la sonde convient pour l'insertion à travers le second tube (2).
